Europäisches Patentamt

European Patent Office

Office européen des brevets

(11) Veröffentlichungsnummer: **0 442 592 A2**

# (12) EUROPÄISCHE PATENTANMELDUNG

(21) Anmeldenummer: 91250035.2

(22) Anmeldetag: 08.02.91

(51) Int. Cl.5: **C12N 15/56**, C12N 15/82, A01H 5/00, C12N 5/10

(30) Priorität: 13.02.90 DE 4004800

(43) Veröffentlichungstag der Anmeldung:
21.08.91 Patentblatt 91/34

(84) Benannte Vertragsstaaten:
AT BE CH DE DK ES FR GB GR IT LI LU NL SE

(71) Anmelder: INSTITUT FÜR GENBIOLOGISCHE FORSCHUNG BERLIN GMBH
Ihnestr.63
W-1000 Berlin 33(DE)

(72) Erfinder: Willmitzer, Lothar, Prof. Dr.
Am kleinen Wannsee 34
W-1000 Berlin 39(DE)
Erfinder: Sonnewald, Uwe, Dr.
Gierkezeile 34
W-1000 Berlin 10(DE)
Erfinder: von Schaeven, Antje, Dr.
Dorfstrasse 67a
W-2355 Belau(DE)

(54) Plasmide zur Herstellung von in Habitus und Ertrag Veränderten Transgenen Pflanzen.

(57) Es werden Plasmide zur Herstellung transgener Pflanzen, sowie diese Pflanzen, die durch den Transfer und die Expression von in den Zuckermetabolismus oder die Zuckerpartitionierung innerhalb einer Pflanze eingreifenden Genen, die auf diesen Plasmiden lokalisiert sind, verändert sind, beschrieben.

Die übertragenen Gene führen in den transgenen Pflanzen zu einer veränderten Verteilung von Photoassimilaten, was zu starken Veränderungen im Habitus wie Größe, Blattform, Internodienabstand und Wurzelbildung sowie zu Veränderungen des Ertrages führt.

Es werden ferner Plasmide beschrieben, die erlauben, fremde Proteine im Vakuolen transgener Pflanzen zu dirigieren.

EP 0 442 592 A2

# PLASMIDE ZUR HERSTELLUNG VON IN HABITUS UND ERTRAG VERÄNDERTEN TRANSGENEN PFLANZEN

Die vorliegende Erfindung betrifft neue Plasmide zur Herstellung transgener Pflanzen, sowie die Pflanzen, die durch den Transfer und die Expression von in den Zuckermetabolismus oder die Zuckerpartionierung innerhalb einer Pflanze eingreifenden Genen, die auf diesen Plasmiden lokalisiert sind, verändert sind.

Das Wachstum, die Entwicklung und der Ertrag einer Nutzpflanze oder einer Zierpflanze hängt von der Energie ab, die diese Pflanze durch die Fixierung von $CO_2$ in Kohlehydrate während der Photosynthese gewinnt. Die primären Orte für die Photosynthese sind das Blatt und zu einem geringeren Ausmaß das Stammgewebe, wohingegen andere Organe von Pflanzen, wie Wurzeln, Samen oder Knollen nicht wesentlich zur Bildung von Photoassimilaten beitragen, sondern im Gegenteil in ihrem Wachstum von der Versorgung durch photosynthetisch aktive Organe abhängig sind. Dieses bedeutet, daß es einen Fluß an photosynthetisch gewonnener Energie von photosynthetisch aktiven Geweben zu photosynthetisch inaktiven Teilen einer Pflanze gibt.

Die photosynthetisch aktiven Gewebe werden als Quellen oder "sources" bezeichnet. Sie werden als Nettoexporteure des fixierten Kohlendioxids definiert. Die photosynthetisch inaktiven Teile einer Pflanze werden als Senken oder "sinks" bezeichnet. Sie werden als Nettoimporteure des photosynthetisch fixierten Kohlendioxids definiert.

Es ist anzunehmen, daß sowohl die effiziente Nutzung von Photosyntheseprodukten als auch ihre Verteilung innerhalb einer Pflanze diese in mehrerer Hinsicht stark beeinflußt. Als Beispiel sei der Habitus von Pflanzen genannt. Sich neu entwickelnde Organe wie sehr junge Blätter oder auch andere Bereiche wie Wurzel und Samen sind völlig von der Photosyntheseleistung der "sources" abhängig. Das bedeutet, daß die Entwicklung solcher Organe von der Verteilung der in den "sources" gebildeten Photoassimilate innerhalb der Pflanze abhängig ist. Die Möglichkeit der Ausbildung von jungen Blättern oder auch der Ausbildung von Wurzeln könnte drastische Auswirkungen auf den Habitus einer Pflanze wie z.B. die Größe einer Pflanze, den Internodienabstand, die Größe und Form eines Blattes, das Aussehen eines Blattes und die Menge und Form der gebildeten Wurzel haben. Weiterhin sollte die Verteilung von Photoassimilaten von ganz entscheidender Bedeutung für den Ertrag einer Pflanze sein. So hat sich die Gesamtphotosyntheseleistung des Weizens in den letzten Jahrzehnten nicht wesentlich verändert, während der für den Menschen erntebare Ertrag der Weizenpflanzen angestiegen ist. Dies ist im wesentlichen darauf zurückzuführen, daß das "sink" - zu "source"-Verhältnis dahingehend geändert wurde, daß die für den Ertrag wichtigen "sinks" wie Samen wesentlich mehr Photoassimilat aufnahmen als andere, für den Ertrag unbedeutende Bereiche der Pflanze, wie z.B. der Stengel. Durch eine in diesem Fall Verkürzung des Halmes konnte somit ein für den Menschen sehr viel günstigeres "sink" - zu "source"-Verhältnis bei Weizen erzielt werden. Dieses unterstreicht die Wichtigkeit der Verteilung von in den primären "sources" gebildeten Photoassimilaten in höheren Pflanzen in Bezug auf sowohl Habitus als auch Ertrag von Pflanzen.

Es ist unbekannt, durch welche biochemischen Mechanismen das Verhältnis von "sink" und "source" reguliert wird.

Neue biotechnologische Verfahren zur genetischen Veränderung dikotyler und monokotyler Pflanzen sind bekannt (Gasser und Fraley, 1989, Science 244 , 1293-1299).

Bei den meisten Pflanzen werden Photoassimilate innerhalb einer Pflanze in Form von Zuckern und zwar präferentiell in der Form von Saccharose verteilt. Die Verteilung der Saccharose zwischen "source" - und "sink" -Geweben erfolgt durch den Transport der Saccharose über das Phloem. Eine der wesentlichen Determinanten für die Stärke eines "sinks" könnte die Entladung des Phloems in den "sink" darstellen. Um eine verstärkte Entladung der Saccharose aus dem Phloem in den 'sink" herbeizuführen, sollte die Saccharose möglichst bald nach Verlassen des Phloems in eine andere chemische Komponente überführt werden, die nicht mehr im chemischen Gleichgewicht mit der Saccharose steht.

Die Veränderungen des Pflanzenhabitus stellen wesentliche Verbesserungen gegenüber bekannten Pflanzen dar. So sollte z.B. eine Verkürzung des Stammes zu weniger windanfälligen Sorten führen. Eine bevorzugte Verteilung der Photoassimilate in erntebare Organe wie Samen, Blätter, z.B. Tabak; Stengel, z.B. Zuckerrohr; Knolle, z.B. Kartoffelknolle; Rübe, z.B. Futterrübe, Zuckerrübe; Frucht, z.B. Tomate, Gerste, Weizen, Sojabohne oder Mais, sollte zu einem höheren Ertrag einer Pflanze führen. Schließlich lassen sich diese Veränderungen auch auf Zier- und Gartenpflanzen übertragen, was zu Pflanzen mit völlig neuem Habitus führt.

Aufgabe der vorliegenden Erfindung ist es nun. Plasmide zur Herstellung von Pflanzen, die in ihrem Habitus wie Größe, Blattform, Internodienabstand und Wurzelausbildung sowie in ihrem Ernteertrag verändert sind, bereitzustellen. Es ist ferner Aufgabe der Erfindung, Pflanzen, die diese Plasmide enthalten, bereitzustellen.

Es wurde nun gefunden, daß Plasmide, auf denen Gene enthalten sind, die in den Zuckermetabolismus oder in die Zuckerpartitionierung eingreifen, nach gezielter Einführung in die Pflanzen, in diesen transgenen Pflanzen exprimiert werden, wobei die kodierten Produkte zu einer veränderten Verteilung von Photoassimilaten in diesen transgenen Pflanzen führen. Dabei konnte überraschend gezeigt werden, daß die Einführung von einzelnen Genen zu starken Veränderungen des Habitus und Ertrages führt.

Eine besonders starke Veränderung des Habitus und Ertrags der Pflanze, wie z.B. der Kartoffel- und Tabakpflanze, konnte mit Plasmiden erreicht werden, die DNA-Sequenzen eines Gens eines Saccharose-modifizierenden Enzyms enthielten, wobei dieses Gen ein Invertase-Gen, wie z.B. das Invertase-Gen Suc2 aus Hefe ist, und wobei die Sequenzen dieser Gene an die regulatorischen Regionen anderer Gene, die eine Expression des Invertase-Gens in pflanzlichen Zellen und Pflanzen sicherstellen, sowie gegebenenfalls an eine DNA-Sequenz, die die Direktion des Invertase-Proteins in Vakuolen der Pflanze und pflanzlicher Zellen sicherstellt, fusioniert sind.

Der DNA-Sequenz des Invertase-Gens kann hierbei gegebenenfalls noch eine weitere DNA-Sequenz vorangestellt werden, die an ein für die Aufnahme in das endoplasmatische Retikulum von Pflanzen und Pflanzenzellen notwendiges Signalpeptid fusioniert ist. Die regulatorischen Regionen sind hierbei Promotoren und Terminationssignale von pflanzlichen Genen. Als Promotoren können Promotoren, die eine annähernd konstitutive Expression, wie z.B. der Promotor des 35 S RNA Cauliflower-Mosaik-Virus, Promotoren, die eine Expression nur in bestimmten Geweben, wie in photosynthetisch aktiven Zellen, wie z.B. der Promotor des ST-LS1 Gens oder in Speicher-"sinks", wie Knollen, Rüben oder Früchten, wie z.B. der Promotor des Klasse I-Patatin-Gens B33 oder in Speicher-"sinks" wie Samen, wie z.B. Samen-spezifisch exprimierte Promotoren, verwendet werden.

Als Signalpeptid kann z.B. das Proteinase-Inhibitor 11-Gen aus Solanum tuberosum verwendet werden, wobei dieses Signalpeptid gegebenenfalls mit Teilen anderer Gene, wie z.B. dem Octopin-Synthase Gen, fusioniert sein kann.

Die DNA-Sequenz, die die Direktion fremder Proteine wie z.B. des Invertaseproteins in Vakuolen der Pflanze und pflanzlicher Zellen sicherstellt, kann z.B. eine DNA-Sequenz des Patatin-Gens pgT5 aus Solanum tuberosum sein. Die DNA-Sequenz, die die Direktion des fremden Proteins in Vakuolen sicherstellt, wird in der Regel zu einem Fusionsprotein mit dem Fremdprotein führen.

Zur Vorbereitung der Einführung fremder Gene in höhere Pflanzen sind eine große Anzahl Klonierungsvektoren verfügbar, die ein Replikationssystem in E. coli und einen Marker, der eine Selektion der transfomierten Zellen erlaubt, beinhalten. Die Vektoren beinhalten z.B. pBR 332, pUC-Serien, M13 mp-Serien, pACY 184 usw. Dementsprechend kann die Sequenz an einer passenden Restriktionsstelle in den Vektor eingefügt werden. Das erhaltene Plasmid wird für die Transformation in E. coli verwendet. Die E. coli Zellen werden in einem geeigneten Nährmedium gezüchtet, anschließend geerntet und lysiert. Das Plasmid wird wiedergewonnen. Als Analysemethoden werden im allgemeinen eine Sequenzanalyse, eine Restriktionsanalyse, Elektrophoresen und weitere biochemisch-molekularbiologische Methoden durchgeführt. Nach jeder Manipulation kann die verwendete DNA-Sequenz gespalten und mit der nächsten DNA-Sequenz verbunden werden. Jede Plasmid-Sequenz kann in den gleichen oder anderen Plasmiden kloniert werden. Je nach Einführungsmethode gewünschter Gene in die Pflanze können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und die linke Begrenzung der Ti- und Ri-Plasmid T-DNA, als Flankenbereich der einzuführenden Gene, verbunden werden.

Die Verwendung von T-DNA für die Transformation von Pflanzenzellen ist intensiv untersucht und ausreichend in EP 120 516; Hoekema, In: The Binary Plant Vector System Offset-drukkerij Kanters B.V., Alblasserdam, 1985, Chapter V; Fraley, et al., Crit. Rev. Plant Sci., 4:1-46 und An et al., EMBO J. (1985) 4: 277-287 beschrieben worden.

Ist die eingeführte DNA erst einmal im Genom integriert, so ist sie dort auch relativ stabil und springt in der Regel nicht mehr heraus. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin oder Chloramphenicol u.a. vermittelt. Der individuell verwendete Marker sollte daher die Selektion transformierter Zellen gegenüber Zellen, denen die eingefügte DNA fehlt, gestatten.

Für die Einführung von DNA in eine pflanzliche Wirtszelle stehen eine Vielzahl von Techniken zur Verfügung. Diese Techniken umfassen die Transformation mit T-DNA unter Verwendung von Agrobacterium tumefaciens oder Agrobacterium rhizogenes als Transformationsmittel, die Fusion, die Injektion oder die Elektroporation sowie weitere Möglichkeiten. Werden für die Transformation Agrobakterien verwendet, muß die einzuführende DNA in spezielle Plasmide kloniert werden und zwar entweder in einen intermediären Vektor oder einen binären Vektor. Die intermediären Vektoren können aufgrund von Sequenzen, die homolog zu Sequenzen in der T-DNA sind, durch homologe Rekombination in das Ti- oder Ri-Plasmid integriert werden. Dieses enthält außerdem die für den Transfer der 7-DNA notwendige Vir-Region. Intermediäre vektoren können sich nicht in Agrobakterien replizieren. Mittels eines Helferplasmids kann der intermediäre Vektor auf Agrobacterium tumefaciens übertragen werden (Konjugation). Binäre Vektoren können sich sowohl in E. coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al., Mol. Gen. Genet. (1978), 163: 181-187). Das als Wirtszelle dienende Agrobakterium soll ein Plasmid, das eine Vir-Region trägt, enthalten. Die Vir-Region ist für den Transfer der T-DNA in die Pflanzenzelle notwendig. Zusätzliche T-DNA kann enthalten sein. Das so transformierte Bakterium wird zur Transformation von Pflanzenzellen benutzt. Für den Transfer der DNA in die Pflanzenzelle können Pflanzen-Explanate zweckmäßiger Weise mit Agrobacterium tumefaciens oder Agrobacterium rhizogenes kultiviert werden. Aus dem infizierten Pflanzenmaterial (z.B. Blattstücke, Stengelsegmente, Wurzeln, aber auch Protoplasten oder Suspensions-kultivierte Zellen) können dann in einem geeigenten Medium, welches Antibiotika oder Biozide zur Selektion enthalten kann, wieder ganze Pflanzen regeneriert werden. Die so erhaltenen Pflanzen können dann auf Anwesenheit der eingeführten DNA getestet werden. Bei der Injektion und Elektroporation sind keine speziellen Anforderungen an die Plasmide gestellt. Es können einfache Plasmide, wie z.B. pUC-Derivate, verwendet werden.

Die transformierten Zellen wachsen innerhalb der Pflanzen in der üblichen Weise. Diese Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften.

## Begriffe und Abkürzungen

### Abkürzungen

| | | |
|---|---|---|
| bp, Kb | = | Basenpaare, Kilobasen |
| d, kd | = | Dalton, Kilodalton |
| DNA | = | Desoxyribonukleinsäure, Träger der genetischen Information |
| SDS | = | Natriumdodecylsulfat |
| Tris | = | Tris(2-aminoäthyl)-amin |
| EDTA | = | Ethylendiamintraessigsäure |

## Begriffe

Elektrophorese
= biochemisches Trennverfahren zur Trennung von Proteinen und Nukleinsäuren nach Größe und Ladung.
Endoplasmatisches Retikulum
= Intrazelluläre membranöse Kanäle, die dem Transport chemischer und biochemischer Substanzen dienen.
Expression
= Aktivität eines Gens
Gen
= Erbfaktor; eine Einheit des Erbguts, Träger der Teilinformation für ein bestimmtes Merkmal. Gene bestehen aus Nukleinsäuren (z.B. DNA, RNA).
Genom
= Gesamtzahl der auf den Chromosomen der Zelle lokalisierten Gene.
Internodien
= Durch Knoten (Nodien) voneinander gesonderte Sproßabschnitte (z.B. Halme, Stengel, usw.) An den Nodien stehen die Blätter.
Internodienabstand
= Abstand der unterschiedlichen Sproßabschnitte von den Nodien.
Klenow-Fragment
= durch Spaltung mit Substilisin erhaltenes 76.000 d großes Fragment der DNA-Polymerase I, Besitzt 5'-3'-Polymerase-und 3'-5'-Exonuklease-Aktivität, aber nicht die 5'-3'-Exonuklease-Aktivität des Holoenzyms.
Klon
= Zellpopulation, die von einer einzigen Mutter stammt.
Die Nachkommen sind genotypisch gleich. Durch Klonierung kann die Einheitlichkeit von Zellinien noch gesteigert werden.
Ligation
= enzymatische Bildung einer Phosphodiesterbindung zwischen 5' -Phosphatgruppen und 3'-Hydroxylgruppen der DNA.
Linker, Polylinker
= synthetische DNA-Sequenz, die eine oder mehrere (Polylinker) Restriktionsschnittstellen in unmittelbarer Reihenfolge enthält.
Northern Blot, Southern Blot
= Transfer und Fixierung elektrophoretisch aufgetrennter RNA oder DNA auf eine Nitrozellulose- oder Nylonmembran
Phänotyp
= Summe der Merkmale, die in einem Organismus im Gegensatz zu seiner Genausstattung (Genotyp) ausgeprägt ist.
Phloem
= Siebteil der Gefäßbündel einer Pflanze. Die Gefäßbündel leiten Wasser mit gelösten Stoffen.

Plasmid

= zusätzlicher, extrachromosomaler DNA-Erbträger in Bakterienzellen (evtl. auch in Eukaryonten), der sich unabhängig vom Bakterienchromosom redupliziert. Das Plasmid kann in andere Wirts-DNA integriert werden.

Promotor

= Kontrollsequenz der DNA-Expression, die die Transkription homologer oder heterologer DNA-Gen-Sequenzen gewährleistet.

Replikation

= Verdopplung der DNA-Sequenz

Restriktionsenzyme

= Restriktionsendonukleasen, die eine Untergruppe der Endodesoxyribonukleasen sind (z.B. EcoRI (Spezifität G↓AATTC und EcoRII↓CC (Ą) GG, zeichnen sich durch eine hohe Spezifität der Substraterkennung aus (↓= Spaltstelle).

Restriktionsstellen

= Spaltstellen, die spezifisch durch Restriktionsenzyme erzeugt werden.

Termination

= letzte Stufe der Proteinsynthese, in der die Polypeptidkette vervollständigt wird.

Transformation

= Einfügung exogener DNA eines Bakterienstammes in eine Empfängerzelle.

Transkription

= Überschreibung der auf der DNA enthaltenen genetischen Information auf eine RNA.

Vektoren

= wirtsspezifische, replizierfähige Strukturen, die Gene aufnehmen und diese auf andere Zellen übertragen. Plasmide können als Vektoren benutzt werden.

Folgende Plasmide wurden bei der Deutschen Sammlung von Mikroorganismen (DSM) in Braunschweig, Bundesrepublik Deutschland, hinterlegt (Hinterlegungsnummer):

Am 12.02.1990
Plasmid p 35 S - Cy-INV (DSM 5785)
Plasmid p 35 S - CW-INV (DSM 5788)
Plasmid p 33 - CW-INV (DSM 5787)
Plasmid pI 700 - CW-INV DSM 5789)
Plasmid p 33 - Cy-INV (DSM 5786)

Am 20.08.1990
Plasmid p35 S - V - INV (DSM 6142)

Beschreibung der Abbildungen

**Fig. 1**
zeigt die Struktur des 5.1 kb großen Plasmids p355-Cy-INV. Das Plasmid enthält folgende Fragmente:

A = Fragment A (529 bp); Beinhaltet den 355 Promotor des Cauliflower-Mosaik-Virus (CaMV). Es enthält ein Fragment, welches die Nukleotide 6909 bis 7437 des CaMV umfaßt.

B = Fragment B (1726 bp): Enthält 23 Nukleotide eines Proteinase Inhibitor II Gens aus der Kartoffel (Nukleotide 923-945), welche über einen Linker von 7 Basenpaaren an das suc2 Gen aus Hefe, umfassend die Nukleotide +64 bis +1765, fusioniert sind.

C = Fragment C (192 bp): Enthält das Polyadenylierungssignal des Gens 3 der 7-DNA des Ti-Plasmids pTiACH5.

Es werden ferner die im Ausführungsbeispiel 1 beschriebenen Schnittstellen gezeigt.

**Fig. 2**
zeigt die Struktur des 7,1 kb großen Plasmids p35S-CW-INV.
Das Plasmid enthält folgende Fragmente:

A = Fragment A (529 bp): Beeinhaltet den 35S Promotor des Cauliflower-Mosaik-Virus (CaMV). Es enthält ein Fragment, welches die Nukleotide 6909 bis 7437 des CaMV umfaßt.

B = Fragment B (1963 bp): Enthält die Nukleotide 923 - 1159 eines Proteinase Inhibitor II Gens aus der Kartoffel, welche über einen Linker an das suc2 Gen aus Hefe, umfassend die Nukleotide +64 bis +1765, fusioniert sind.

C = Fragment C (192 bp): Enthält das Polyadenylierungssignal des Gens 3 der T-DNA des Ti-Plasmids pTiACH5.

Es werden ferner die im Ausführungsbeispiel 2 beschriebenen Schnittstellen gezeigt.

**Fig. 3**
zeigt die Struktur des 7,0 kb großen Plasmids p33-CW-INV.
Das Plasmid enthält folgende Fragmente:

A = Fragment A (1526 bp): Beinhaltet das DraI-DraI Fragment (Position -1512 bis Position +14) der Promotor Region des Patatin Gens B33.

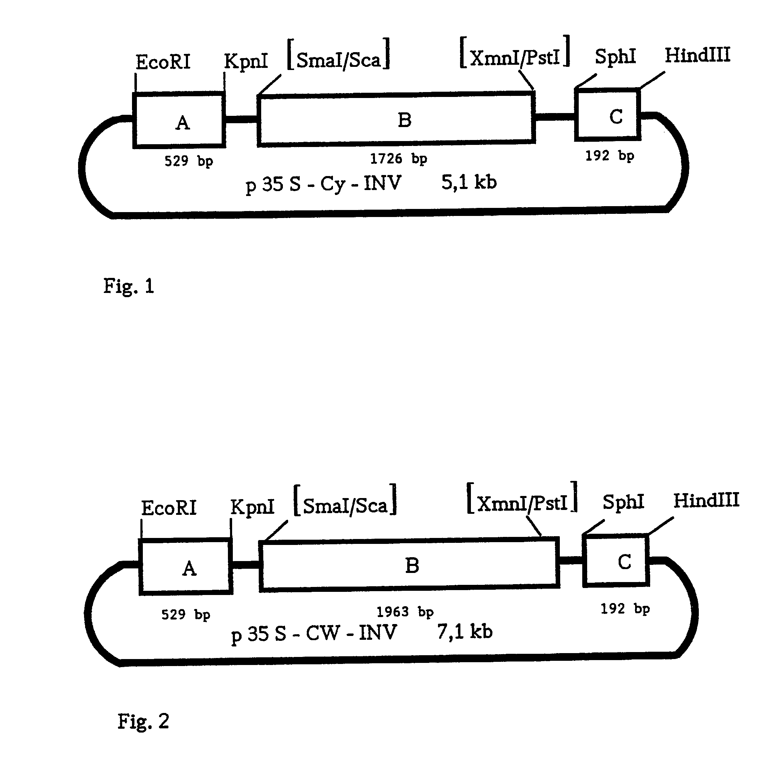

B und C = Fragment B (1963 bp) und C (192 bp): Sie entsprechen den Fragmenten B und C im Plasmid p35S-6W-INV (Fig. 2).

Es werden ferner die im Ausführungsbeispiel 3 beschriebenen Schnittstellen gezeigt.

**Fig. 4**
zeigt die Struktur des 8,4 kb großen Plasmids p1700-CW-INV.

Das Plasmid enthält folgende Fragmente:

A =        Fragment A (1585 bp): Enthält
           das EcoRI-MboII Fragment des
           ST-LS1-Gens aus der Kartoffel.
B und C =  Fragment B (1963 bp) und C
           (192 bp): Entsprechen den Fragmenten B und C im Plasmid
           p35S-CW-INV (Fig. 2).

Es werden ferner die im Ausführungsbeispiel 4
beschriebenen Schnittstellen gezeigt.

**Fig. 5**
zeigt die Struktur des 6,8 kb großen Plasmids p33-
Cy-INV.
Das Plasmid enthält folgende Fragmente:

A =        Fragment A (1526 bp): Enthält
           das DraI-DraI Fragment (Position
           -1512 bis Position +14) der Promotor Region des Patatin Gens
           B33.
B und C =  Fragmente B (1726 bp) und C
           (192 bp): Entsprechen den Fragmenten B und C im Plasmid
           p35S-Cy-INV (Fig. 1).

Es werden ferner die im Ausführungsbeispiel 5
beschriebenen Schnittstellen gezeigt.

**Fig. 6**
zeigt die Struktur des 6,3 kb großen Plasmids p35
S-V-INV.
Das Plasmid enthält folgende Fragmente:

A =   Fragment A (529 bp): Enthält den 35S
      Promotor des Cauliflower-Mosaik-Virus
      (CaMV).
B =   Fragment B (2898 bp): Enthält die Nukleotide +707 bis +1895 der Sequenz
      des genomischen Patatinklons pgT5,
      einen Linker der Sequenz AGCTTTC
      und das Suc 2 Gen aus Hefe
      (Nukleotide +64 bis +1765).
C =   Fragment C (192 bp): Enthält das Polyadenylierungssignal des Gens 3 der
      T-DNA des Ti-Plasmids pTiACH5
      (Nukleotide 11749-11939).

Es werden ferner die im Ausführungsbeispiel 6
beschriebenen Schnittstellen gezeigt.

**Fig. 7**
zeigt das Gel für den in situ Nachweis der Invertaseaktivität in Blattextrakten aus fünf unabhängigen
transgenen Tabakpflanzen (Spur 1-5) sowie die Abwesenheit einer solchen Aktivität in nicht-transformierten Pflanzen (Spur W38)

A =    Gelbereich der reduzierenden Zucker.

Die schwarzen Flecken der Spur 1-5
zeigen die Anwesenheit reduzierender
Zucker (Invertaseaktivität) im Gegensatz zur Kontrolle (Spur W38)
B =    Gelbereich der Proteinfraktion.

**Fig. 8**
zeigt die Anzahl, die Größenverteilung
(Frischgewicht) und das Gesamtfrischgewicht der
Knollen von zwei mit dem Plasmid p33-CW-INV
transformierten Kartoffelpflanzen (linke Hälfte,
Pflanzen B33-CW-IN-4 und B33-CW-IN-1), sowie
zwei unter den exakt gleichen Bedingungen gewachsenen und nicht transformierten Kontrollpflanzen (rechte Hälfte, Pflanzen control-1 und control-
2). Jeder vertikale Balken steht für eine Knolle,
deren Gewicht in g oberhalb des Balkens angegeben ist. Ebenfalls angegeben ist das Gesamtgewicht (total) in g.

**Fig. 9**
zeigt das Gel für den in situ Nachweis, der vom
Plasmid p35S-V-INV kodierten Invertaseaktivität in
den Vakuolen der mit diesem Plasmid transformierten Tabakpflanzen.

Spur 1: Protoplasten untransformierter Tabakpflanzen; Spur 2: Vakuolen untransformierter Tabakpflanzen; Spur 3: Protoplasten transgener Tabakpflanzen; Spur 4: Vakuolen transgener Tabakpflanzen.

In jeder Spur wurden vergleichbare Mengen
Protoplasten bzw. Vakuolen, die über die α-
Mannosidase-Aktivität abgeglichen worden waren,
aufgetragen. Die schwarzen Flecken in Spur 3 und
4 zeigen die Invertaseaktivität über die Bildung
reduzierender Zucker an. Die Intensität in beiden
Spuren ist gleich hoch, was die ausschließliche
Lokalisierung der Invertase in den Vakuolen beweist. Diese Invertaseaktivität ist in den Protoplasten und Vakuolen nicht-transformierter Tabakpflanzen (Spur 1 und 2) nicht enthalten.

Zum besseren Verständnis der dieser Erfindung zugrundeliegenden Ausführungsbeispiele wird
vorab eine Aufstellung aller für diese Versuche
notwendigen und an sich bekannten Verfahren gegeben:

1. Klonierungsverfahren
Zur Klonierung wurden die Vektoren pUC18/19
und pUC118 (Yanisch-Perron et., Gene (1985),
33, 103-119) und pMPK110 (Eckes, Dissertation,
Universität zu Köln (1984)) benutzt.

Für die Pflanzentransformation wurden die
Genkonstruktionen in den binären Vektor BIN19
(Bevan, Nucl. Acids Res. (1984), 12, 8711-
8720) kloniert.

2. Bakterienstämme
Für die pUC-Vektoren wurden die E. coli-Stämme BMH71-18 (Messing et al., Proc. Natl. Acad. Sci. USA (1977), 24, 6342-6346) oder TB1 benutzt.

Für den Vektor BIN19 und pMPK 110 wurde ausschließlich der E. coli-Stamm TB1 benutzt. TB1 ist ein Rekombinations-negatives, Tetracyclin-resistentes Derivat des Stammes JM101 (Yanisch-Perron et al., Gene (1985), 33, 103-119). Der Genotyp des TB1-Stammes ist (Bart Barrel, pers. Mitteilung): F' (traD36, proAB, lacI, lacZΔM15), Δb(lac, pro), SupE, thiS, recA, Sr1 :: Tn10(Tc$^R$).

Die Pflanzentransformation wurde mit Hilfe des Agrobacterium tumefaciens-Stammes LBA4404 (Bevan, M., Nucl. Acids Res. 12, 8711-8721, (1984): BIN19 Derivat) durchgeführt.

3. Transformation von Agrobacterium tumefaciens

Bei Bin19-Derivaten erfolgt die Einführung der DNA in die Agrobakterien durch direkte Transformation nach der Methode von Holsters et al., (Mol Gen. Genet. (1978), 163, 181-187). Die Plasmid-DNA transformierter Agrobakterien wurde nach der Methode von Birnboim und Doly (Nucl. Acids Res. (1979), 7, 1513-1523) isoliert und nach geeigneter Restriktionsspaltung gelelektrophoretisch aufgetrennt.

4. Pflanzentransformation

A) Tabak: 10 ml einer unter Selektion gewachsenen Übernachtkultur von Agrobacterium tumefaciens wurden abzentrifugiert, der Überstand verworfen, und die Bakterien im gleichen Volumen Antibiotika-freien Mediums resuspendiert. In einer sterilen Petrischale wurden Blattscheiben steriler Pflanzen (ca. 1 cm$^2$), denen die Mittelrippe entfernt wurde, in dieser Bakteriensuspension gebadet. Anschließend wurden die Blattscheiben in Petrischalen, die MS-Medium (nach Murashige und Skoog, Physiologia Plantarum (1962), 15, 473-497) mit 2 % Saccharose und 0,8 % Bacto-Agar enthalten, dicht ausgelegt. Nach 2-tägiger Inkubation bei 25° C im Dunkeln wurden sie auf MS-Medium übertragen, welches 100 mg/l Kanamycin 500 mg/l Claforan, 1 mg/l Benzylaminopurin (BAP), 0,2 mg/l Naphthylessigsäure (NAA) und 0,8 % Bacto-Agar enthielt. Wachsende Sprosse wurde auf hormonfreies MS-Medium mit 250 mg/l Claforan überführt.

B) Kartoffel: 10 kleine, mit einem Skalpell verwundete Blätter einer Kartoffel-Sterilkultur wurden in 10 ml MS-Medium mit 2 % Saccharose gelegt, welches 30 - 50μl einer unter Selektion gewachsenen Agrobacterium tumefaciens-Übernachtkultur enthielt. Nach 3-5 minütigem, leichtem Schütteln wurden die Petrischalen bei 25°C im Dunkeln inkubiert. Nach 2 Tagen wurden die Blätter auf MS-Medium mit 1,6 % Glukose, 2 mg/l Zeatinribose, 0,02 mg/l Naphthylessigsäure, 0,02 mg/l Giberellinsäure, 500 mg/l Claforan, 50 mg/l Kanamycin und 0,8 % Bacto-Agar ausgelegt. Nach einwöchiger Inkubation bei 25°C und 3000 Lux wurde die Claforankonzentration im Medium um die Hälfte reduziert. Eine weitere Kultivierung erfolgte nach bekannten Methoden (Rocha-Sosa et. al., EMBO J. 8, 29 (1989).

5. Analyse genomischer DNA aus transgenen Pflanzen

Die Isolierung genomischer Pflanzen-DNA erfolgte nach Rogers und Bendich (Plant Mol. Biol. (1985), 5, 69-76).

Für die DNA-Analyse wurden 10 - 20 μg DNA nach geeigneter Restriktionsspaltung mit Hilfe von "Southern Blots" auf Integration der zu untersuchenden DNA-Sequenzen analysiert.

6. Analyse der Gesamt-RNA aus transgenen Pflanzen

Die Isolierung pflanzlicher Gesamt-RNA erfolgte nach Logemann et al. (Analytical Biochem. (1987), 163, 16-20).

Für die Analyse wurden je 50 μg Gesamt-RNA mit Hilfe von "Northern Blots" auf die Anwesenheit der gesuchten Transkripte untersucht.

7. Proteinextraktion

Zur Extraktion von Gesamtprotein aus Pflanzengewebe wurden Gewebestücke in Proteinextraktionspuffer (25 mM Natriumphosphat pH 7,0, 2 mM Natriumbisulfit, 2mM Phenylmethylsulfonylfluorid (PMSF)), unter Zusatz von 0,1 % (w/v) unlöslichem Polyvinylpyrrolidon (PVP) homogenisiert.

Nach Filtrieren durch Zellstoff wurden Zelltrümmer für 20 Minuten bei 10000 Umdrehungen pro Minute abzentrifugiert und die Proteinkonzentration des Überstandes nach der Methode von Bradford (Anal. Biochem. (1976)/ 72, 248-254) bestimmt.

8. Nachweis von Fremdproteinen mit Hilfe immunologischer Verfahren (Western-Blot)

Proteinextrakte wurden mittels Gelelektrophorese in SDS-PAGE (Natriumdodecylsulfat-Polyacrylamid) Gelen nach Molekulargewicht getrennt. Nach SDS-PAGE wurden Proteingele für 15-30 Minuten in Transfer Puffer für Graphitelektroden (48 g/l Tris, 39 g/l Glycin, 0,0375 % SDS, 20 % Methanol) äquilibriert und anschließend im Kühlraum auf Nitrozellulose-Filter transferiert und mit 1,3 mA/cm$^2$ für 1-2 Stunden getrennt). Der Filter wurde für 30 Minuten mit 3 % Gelatine in TBS-Puffer (20 mM Tris/HCl pH 7,5, 500 mM NaCl) abgesättigt, anschließend wurde der Filter für 2 Stunden mit dem entsprechenden Antiserum in geeigneter Verdünnung (1:1000 - 10000 in TBS Puffer) bei Raumtemperatur inkubiert. Danach wurde der Filter jeweils für 15 Minuten mit TBS-, TTBS- (TBS-Puffer mit 0,1 % Tween 20) und TBS-Puffer gewaschen. Nach dem Waschen wurde der Filter für 1 Stunde bei Raumtemperatur mit Alkalische Phosphatase konjugierten Goat-anti-Rabbit (GAR)-Antikörpern (1:7500 in TBS) inkubiert. Anschließend wurde der Filter wie obenstehend beschrieben gewaschen und in AP-Puffer (100 mM Tris/HCl pH 9.5, 100 mM NaCl, 5 mM $MgCl_2$) äquilibriert. Die Alkalische Phosphatase Reaktion wurde durch Substratzugabe von 70 $\mu$l 4-Nitrotetrazolium (NBT)-Lösung (50 mg/ml NBT in 70 % Dimethylformamid) und 35 $\mu$l 5-Brom-4-Chlor-3-Indolylphosphat (BCIP) (50 mg/ml BCIP in Dimethylformamid) in 50 ml AP-Puffer gestartet. Nach 5 Minuten konnten in der Regel erste Signale beobachtet werden. Die Reaktion kann durch Überführen des Filters in Stop-Lösung (20 mM Tris/HCl pH 8,0 mit 5 mM EDTA) beendet werden. Die Reaktion wurde im Dunkeln durchgeführt.

9. Nachweis der Invertase-Aktivität

Saure Invertase spaltet Saccharose in Glucose und Fructose Die Enzymaktivität von saurer Invertase kann in Pflanzenproteinextrakten nach Auftrennung in SDS-Polyacrylamidgelen nachgewiesen werden.

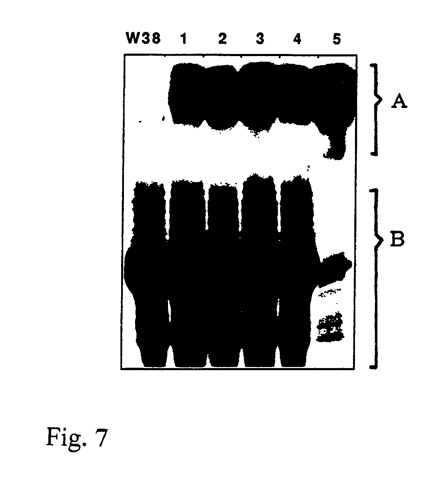

Das Gesamtprotein wurde aus Pflanzen wie unter Punkt 7 beschrieben extrahiert und mit 2x nativem SB-Puffer (125 mM Tris/HCl pH 6,8, 10 % 2-Mercaptoethanol, 20 % Glycol, 0,004 % Bromphenolblau) versetzt und auf 0,2 %ige SDS-Gele gegeben. Die Ansätze wurden vor Auftrennung in den SDS-Polyacrylamidgelen nicht durch Erhitzen denaturiert. Nach elektrophoretischer Trennung wurden die Gele kurz in Wasser gespült und für 1 Stunde bei 30°G in Saccharose-Lösung (0,1 M Saccharose, 0.1 M Natriumacetat pH 5,0) inkubiert. Anschließend wurde überschüssige Saccharose durch mehrmaliges Waschen (3x 5 Minuten) mit Wasser entfernt. Der Nachweis von reduzierenden Zukkern erfolgte durch Kochen der Gele in TPTC-Reaktionslösung (0,1 % 2,3,5-Triphenyltetrazolumchlorid in 0,5 N Natronlauge) für 5 - 10 Minuten im Mikrowellenherd. Die Reaktion wurde in 10 %iger Essigsäure abgestoppt. Die Gele wurden nach dem Wässern getrocknet. Eine intensive Rotfärbung in den Gelen zeigte die Anwesenheit von reduzierenden Zuckern (s. Fig. 7, unter A als schwarze Flecken sichtbar).

10. Isolierung von Vakuolen aus transgenen und nicht-transgenen Tabakpflanzen

Protoplasten wurden aus drei bis vier Wochen alten, steril angezogenen Tabakpflanzen nach bekannter Methode hergestellt (Damm und Willmitzer, Mol. Gen. Genetics 217, 15-20 (1988)). Anschließend wurden aus ca. 10 Millionen Protoplasten Vakuolen nach bekannter Methode freigesetzt (Boller und Kende, Plant Physiology 63, 1123-1132 (1979)). Die Reinheit der Vakuolen wurde mikroskopisch und durch Bestimmung der $\alpha$-Mannosidase-Aktivität bestätigt (Van der Wilden et al., Plant Physiology 66, 390-394 (1980)). Der Invertase-Aktivitätsnachweis erfolgte gelelektrophoretisch nach $\alpha$-Mannosidase-Abgleich.

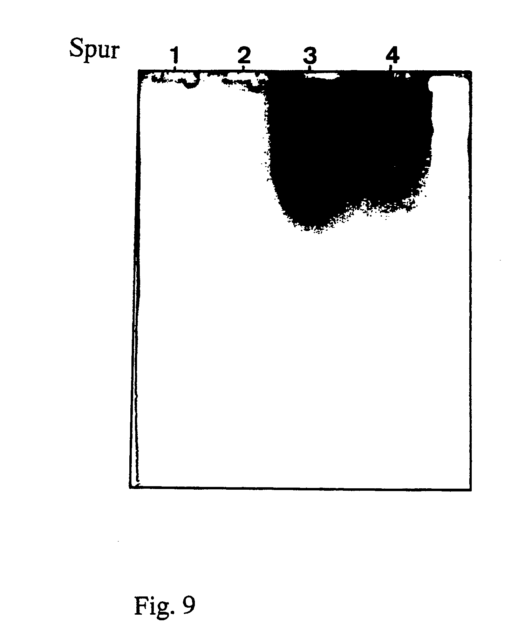

In Fig. 9 ist dargestellt, daß die vakuoläre Fraktion eine vergleichbare Invertase-Aktivität, wie die zur Vakuolen-Isolierung eingesetzten Protoplasten, enthält. Das Ergebnis zeigt, daß die subzelluläre Verteilung der Invertase dem des vakuolären Markerenzyms $\alpha$-Mannosidase entspricht.

Ausfuhrungsbeispiel 1

Herstellung von Plasmid p35S-Cy-INV und Einführung des Plasmids in das pflanzliche Genom des Tabaks und der Kartoffel

Saccharose wird durch das Enzym Invertase in die beiden Hexosen Glucose und Fructose gespalten. Diese beiden Hexosen stehen nicht in einem chemischen Gleichgewicht mit der Saccharose und fuhren daher nicht zu einem Ruckstau in Bezug auf die Entladung des Phloems bezüglich der Saccharose. Eine DNA-Sequenz aus Hefe, die für das suc2 Gen kodiert, wird mit einem eine konstitutiv Expression bewirkenden Promotor der 35S RNA des Cauliflower-Mosaik-Virus sowie einem pflanzlichen Terminationssignal versehen. Das pflanzliche Terminationssignal beinhaltet das 3'-Ende der Poly-A Seite des Octopin-Synthase Gens. Das Plasmid p35S-Cy-INV besteht aus den drei Fragmenten A, B und C, die in die Schnittstellen für Restriktionsenzyme des Polylinkers von pUC18 kloniert wurden (s. Fig. 1).

Das Fragment A beinhaltet den 35S Promotor des Cauliflower-Mosaik-Virus (CaMV). Es enthält ein Fragment, welches die Nukleotide 6909 bis 7437 des CaMV (Franck et al., (1980) Cell 21, 285-294) umfaßt und wurde als EcoRI-KpnI Fragment aus dem Plasmid pDH51 (Pietrzak et al (1986) Nucleic Acids Res. 14, 5857-5868) isoliert und zwischen die Eco RI-KpnI Schnittstellen des Plasmids pUC18 kloniert.

Das Fragment B enthält 23 Nukleotide eines Proteinase Inhibitor II Gens aus Kartoffel (Solanum tuberosum) (Nukleotide 923-945, Keil et al (1986), Nucleic Acids Res. 14, 5641-5650), welche über einen Linker von 7 Basenpaaren mit der Sequenz AGCTTTC an das suc2 Gen aus Hefe, umfassend die Nukleotide +64 bis +1765 (Taussig und Carlson (1983) Nucleic Acids Res. 11, 1943-1954), fusioniert ist.

Das Fragment B wurde als Sca/XmnI Fragment zwischen die SmaI/PstI Schnittstellen des Polylinkers von pUC18 gesetzt, wobei vor der Ligation die 3'-überhängenden Enden der PstI Schnittstelle durch Inkubation mit T4-DNA Polymerase stumpf gemacht worden sind.

Das Fragment C enthält das Polyadenylierungssignal des Gens 3 der T-DNA des Ti-Plasmids pTiACH5 (Gielen et al. (1984); EMBO J. 3, 835-846), Nukleotide 11749 - 11939, welches als PvuII-HindIII Fragment aus dem Plasmid pAGV 40 (Herrera-Estrella et al (1983) Nature 303, 209-213) isoliert worden ist und nach Addition von SphI-Linkern an die PvuII-Schnittstelle zwischen die SphI-HindIII Schnittstellen des Polylinkers von pUC18 kloniert worden war. Das Plasmid p 35 S - Cy - INV hat eine Größe von 5,1 kb (s. Fig. 1).

Das die Fragmente A, B und C umfassende Teilstück des Plasmids p35S-Cy-INV wird in binäre Vektoren eingeführt und mit Hilfe des Agrobacteriumsystems in Tabak- und Kartoffelpflanzen eingeführt. Aus transformierten Zellen wurden intakte und fertile Pflanzen regeneriert. Die Analyse der regenerierten Pflanzen zeigte in allen analysierten Geweben (Blatt, Stamm) eine Invertaseaktivität, die in nicht-transformierten Pflanzen fehlte. Durch immunologische Verfahren konnte nachgewiesen werden, daß es sich bei dieser Invertase um die Hefeinvertase handelt. Diese Invertase ist im Cytosol bzw. Cytoplasma lokalisiert. Es wurden somit transgene Tabak- und Kartoffelpflanzen hergestellt, die in allen Organen und Zellen eine neue Invertaseaktivität enthielt, die von dem in diese Pflanzen eingeführten Hefeinvertasegen stammt. Die regenerierten Tabak- und Kartoffelpflanzen zeigten einen deutlichen Unterschied in Bezug auf nicht-transformierte Pflanzen. So wiesen die Blätter eine Variation der Grünfärbung auf. Weiterhin zeigten einzelne transformierte Pflanzen unterschiedlich starke Zwergwüchsigkeit, was im wesentlichen auf eine Verkürzung des Internodienabstandes bei gleichbleibender Blattzahl zurückzuführen ist. Ferner konnte beobachtet werden, daß die jungen Blätter leicht nach innen gerollt waren.

Ausführungsbeispiel 2

Herstellung des Plasmids p35S-CW-INV und Einführung des Plasmids in das pflanzliche Genom des Tabaks und der Kartoffel

In analoger Verfahrensweise wie unter Beispiel 1 beschrieben, jedoch mit der Modifikation, daß vor die kodierende Sequenz des Invertase-Gens ein für die Aufnahme in das endoplasmatische Retikulum notwendiges Signalpeptid eines pflanzlichen Gens (Proteinase Inhibitor II Gen aus Kartoffel (Solanum tuberosum), Keil et al, 1986) fusioniert wurde, wurde das Plasmid p35S-CW-INV hergestellt. Das Plasmid p35S-CW-INV hat eine Größe von 7,1 kb und besteht aus den drei Fragmenten A, B und C, die in die an gegebenen Schnittstellen für Restriktionsenzyme des Polylinkers von pMPK110 kloniert wurden (s. Fig. 2).

Das Fragment A beinhaltet den 355 Promotor des Cauliflower-Mosaik-Virus (CaMV). Es enthält ein Fragment, welches die Nukleotide 6909 bis 7437 des CaMV (Franck et al (1980) Cell 21, 285-294) umfaßt und wurde als EcoRI-KpnI Fragment aus dem Plasmid pDH51 (Pietrzak et al (1986) Nucleic Acids Res. 14, 5857-5868) isoliert und zwischen die EcoRI-KpnI Schnittstellen des Plasmids pMPK110 kloniert.

Das Fragment B enthält die Nukleotide 923 - 1159 eines Proteinase Inhibitor II Gens aus der Kartoffel (Solanum tuberosum) (Keil et al (1986) Nucleic Acids Res. 14, 5641-5650). welcher über einen Linker mit der Sequenz ACC GAA TTG GGG ATC CCA GCT TTC an das suc2 Gen aus Hefe, welches die Nukleotide +64 bis +1765 (Taussig und Carlson (1983) Nucleic Acids Res. 11, 1943-1954) umfaßt, fusioniert ist. Dadurch wird ein für die Aufnahme von Proteinen in das endoplasmatische Retikulum notwendiges Signalpeptid eines pflanzlichen Proteins N-terminal an die Invertasesequenz fusioniert. Das Fragment B wurde als Sca/XmnI Fragment zwischen die SmaI/PstI Stellen des Polylinkers von pMPK110 gesetzt, wobei vor der Ligation die 3'-überhängenden Enden der PstI Schnittstelle durch Inkubation mit T4 DNA Polymerase stumpf gemacht worden sind.

Das Fragment C enthält das Polyadenylierungssignal des Gens 3 der T-DNA des Ti-Plasmids pTiACH5 (Gielen et al (1984) EMBO J. 3, 835-846, Nukleotide 11748 - 11939), welches als PvuII-HindIII Fragment aus dem Plasmid pAGV 40 (Herrera-Estrella et al (1983) Nature 303, 209-213) isoliert worden ist und nach Addition von SphI-Linkern an die PvuII-Schnittstelle zwischen die SphI-HindIII Schnittstellen des Polylinkers von pMPK110 kloniert worden war (s. Fig. 2).

Das die Fragmente A, B und C umfassende Teilstück des Plasmids p35S-CW-INV wurde in analoger Verfahrensweise wie im Beispiel 1 beschrieben in die Pflanzen eingeführt. Die Analyse der transgenen Pflanzen mittels Western-Blot und Aktivitätstest zeigte, daß die vom suc2 Gen kodierte Invertase nunmehr im extrazellulären Raum lokalisiert ist. Transgene Tabak-und Kartoffelpflanzen, die dieses chimäre Gen enthalten, zeigen zusätzlich zu einer Stauchung der Pflanzen aufgrund eines verringerten Internodienabstands einen neuen Blattphänotyp, der von der Ausbildung eines Mosaikmusters aus grünen und chlorotischen Bereichen bis hin zu der Ausbildung von nekrotischen Bereichen reicht. Weiterhin weisen sie stark reduzierte Wurzelbildung auf.

Ausführungsbeispiel 3

Herstellung des Plasmids p33-CW-INV und Einführung des Plasmids in das pflanzliche Genom der Kartoffel

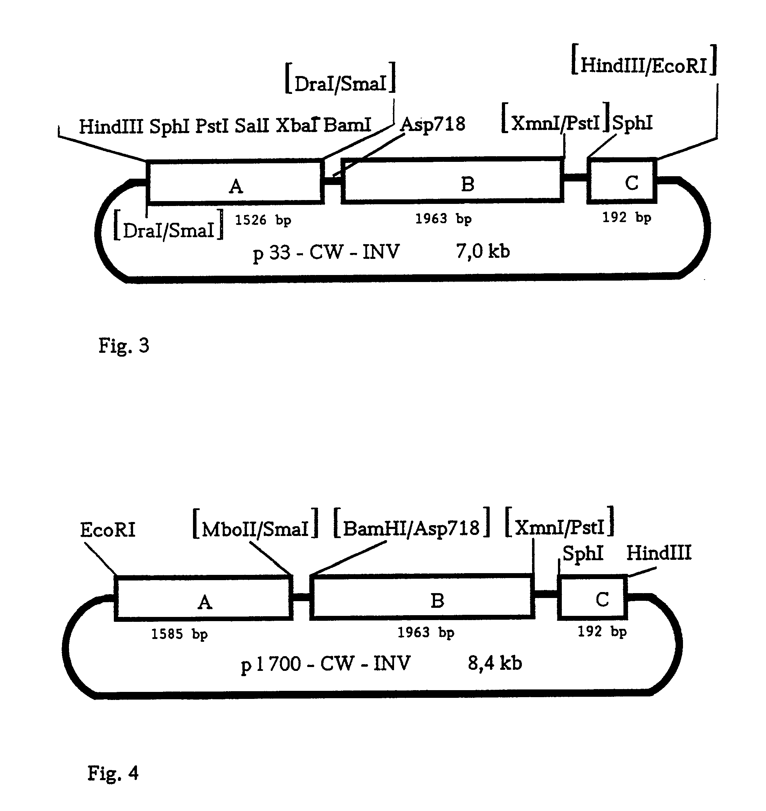

In analoger Verfahrensweise wie unter Beispiel 2 beschrieben, jedoch unter Austausch des 35S Promotors gegen den Promotor des Klasse I Patatin-Gens B33 (Rocha-Sosa et al (1989) EMBO J. 8, 23-29), wurde das Plasmid p33-CW-INV hergestellt. Das Plasmid p33S-CW-INV hat eine Größe von 7,0 kb und besteht aus den drei Fragmenten A, B und C,die in die Schnittstellen für Restriktionsenzyme des Polylinkers von pUC118 kloniert wurden (s. Fig. 3).

Das Fragment A enthält das DraI-DraI Fragment (Position -1512 bis Position +14) der Promotor Region des Patatin Gens B33 (Rocha-Sosa et al (1989) EMBO J, 8, 23-29), welches in die SmaI-Stelle des Polylinkers von pUC118 kloniert wurde.

Die Fragmente B und C entsprechen den Fragmenten B und C im Plasmid p35S-CW-INV (s. Fig. 2). Zur Klonierung des Fragments B und C wurde das Plasmid p35S-CW-INV mit Asp718 (partiell) und HindIII verdaut, die zurückstehenden Enden der HindIII-Schnittstelle mit DNA Polymerase (Klenow Fragment) aufgefüllt und das die beiden intakten Fragmente B und C enthaltende Fragment mittels Gelelektrophorese von anderen Fragmenten abgetrennt. Dieses Fragment wurde sodann in der oben angegebenen Orientierung zwischen die mit DNA Polymerase (Klenow Fragment) partiell mit G + A aufgefüllte EcoRI Schnittstelle und die Asp718 Schnittstelle kloniert. Durch die partielle Auffüllung wurde die HindIII-Schnittstelle erhalten.

Das Plasmid p33-CW-INV wurde in analoger Verfahrensweise wie im Beispiel 1 beschrieben in die Pflanzen eingeführt. Bei der Kartoffel führte das chimäre Gen zu einer Knollenspezifischen Expression der Invertase.

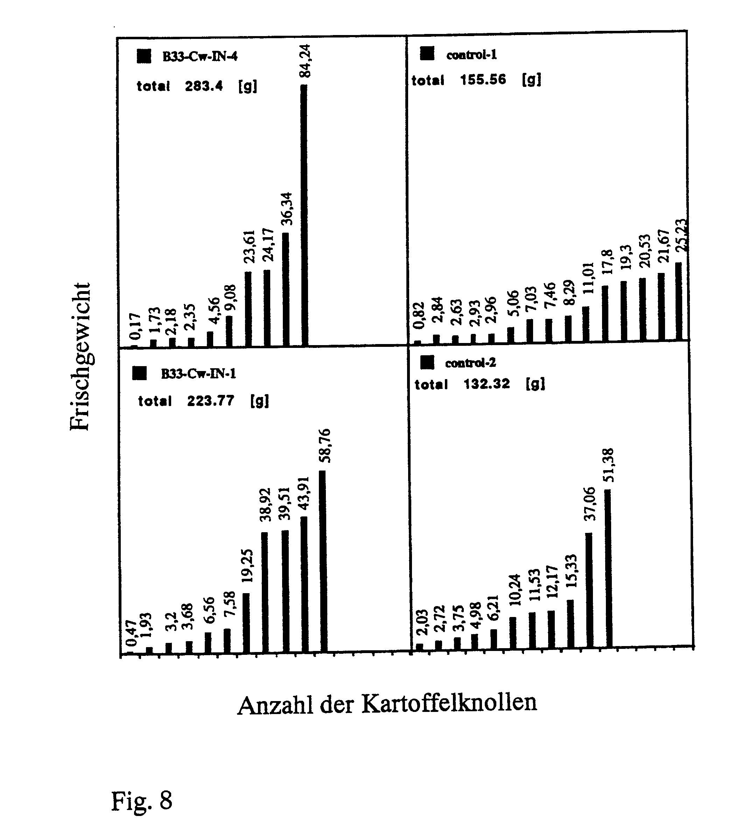

Zwei transgene Kartoffelpflanzen der Sorte Desiree, die mit dem Plasmid p33-CW-INV transformiert worden waren, wurden unter Gewächshausbedingungen ausführlich auf Habitus und Ertrag mit Kontrollpflanzen, die nicht transformiert worden waren, verglichen. Der signifikanteste Unterschied aber ergab sich überraschenderweise bei dem Knollenertrag. So wiesen die beiden mit dem Plasmid p33-CW-INV transformierten Pflanzen unter Gewächshausbedingungen einen Knollenertrag von 283 bzw. 223 g auf, während die beiden Kontrollpflanzen lediglich einen Knollenertrag von 155 bzw. 132 g Frischgewicht aufwiesen. Die Bestimmung des Trockengewichtes sowie des Gesamtstärkegehaltes der Knollen zeigte gleiche relative Zunahmen bei den mit dem Plasmid p33-CW-INV transformierten Pflanzen im Vergleich zu den Kontrollpflanzen. Dieses bedeutet, daß die Einführung und die knollenspezifische Expression des Plasmids p33-CW-INV in transgenen Kartoffelpflanzen den Ertrag dieser Pflanzen um 50-100 % gesteigert hat (siehe Fig. 8). Bezüglich der Größenverteilung der Knollen enthielten die mit dem Plasmid p33-CW-INV transformierten Pflanzen einen wesentlich höheren Anteil an größeren Knollen (siehe Fig. 8). Dies bedeutet, daß sich durch die Einführung und - Expression des Plasmids p33-CW-INV nicht nur der Gesamtknollenertrag bei Kartoffelpflanzen signifikant steigern läßt, sondern auch die Größe der einzelnen Kartoffelknollen signifikant erhöhen läßt.

Ausführungsbeispiel 4

Herstellung des Plasmids p1700-CW-INV und Einführung des Plasmids in das pflanzliche Genom des Tabaks und der Kartoffel

In analoger Verfahrensweise, wie unter Beispiel 2 beschrieben, jedoch unter Austausch des 35S Promotors gegen den blattspezifischen Promotor des ST-LS1 Gens (Stockhaus et al (1987) Proc. Natl. Sci U.S.A. 84, 7943-7947). wurde das Plasmid p1700-CW-INV hergestellt.

Das Plasmid p1700-CW-INV hat eine Größe von 8,4 kb und besteht aus den drei Fragmenten A, B und C, die in die Schnittstellen für Restriktionsenzyme des Polylinkers von pMPK110 kloniert wurden (s. Fig. 4).

Das Fragment A enthält das EcoRI-MboII Fragment des ST-LS1-Gens aus Kartoffel. Die Position der MboII-Seite, in Bezug auf die publizierte Sequenz, (Eckes et al (1986) Mol. Gen. Genetics 205, 14-22) liegt bei Position 1585 (Position +1 bis Position +1585). Dieses Fragment wurde zwischen die EcoRI-SmaI Schnittstellen des Polylinkers von PUC18 kloniert, wobei das überhängende 3'-Ende der MboII Schnittstelle vorher durch T4-DNA Polymerase stumpf gemacht worden war.

Die Fragmente B und C entsprechen den Fragmenten B und C im Plasmid p35S-CW-INV (s. Fig. 2). Zur Klonierung der Fragmente B und C wurde das Plasmid p35S-CW-INV mit Asp 718 partiell verdaut, die zurückstehenden 3' - Enden mit DNA Polymerase (Klenow Fragment) aufgefüllt und das Plasmid mit HindIII nachgespalten. Das die beiden intakten Fragmente B und C enthaltende Fragment wurde mittels Gelelektrophorese von anderen Fragmenten abgetrennt, gereinigt und zwischen die BamHI-HindIII Schnittstellen des Polylinkers von pMPK 110 kloniert. Die BamHI Schnittstelle war dabei vorher durch Auffüllen mit DNA Polymerase I stumpf gemacht worden.

Das die Fragmente A, B und C enthaltende Teilstück des Plasmids p1700-CW-INV wurde in analoger Verfahrensweise, wie im Beispiel 1 beschrieben, in die Pflanzen eingeführt.

## Ausführungsbeispiel 5

Herstellung des Plasmids p33-Cy-INV und Einführung des Plasmids in das pflanzliche Genom des Tabaks und der Kartoffel

In analoger Verfahrensweise wie unter Beispiel 1 beschrieben, jedoch unter Austausch des 35S Promotors gegen den Promotor des Klasse I Patatin-Gens B33 (Rocha-Rosa et al (1989) EMBO J. 8, 23-29), wurde das Plasmid p33-Cy-INV hergestellt.

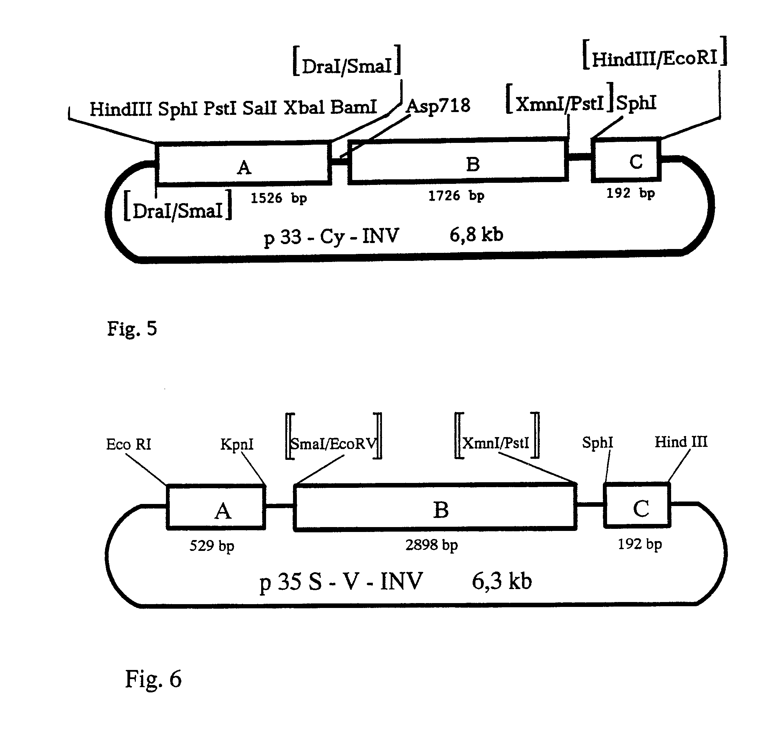

Das Plasmid p33-Cy-INV hat eine Größe von 6,8 kb und besteht aus den drei Fragmenten A, B und C, die in die Schnittstellen für Restriktionsenzyme des Polylinkers von pUC118 kloniert wurden (s. Fig. 5).

Das Fragment A enthält das DraI-DraI Fragment (Position -1512 bis Position +14) der Promotor Region des Patatin Gens B33 (Rocha-Sosa et al (1989) EMBO J. 8. 23-29) , welches in die SmaI-Stelle des Polylinkers von pUC118 kloniert wurde.

Die Fragmente B und C entsprechen den Fragmenten B und C im Plasmid p35S-Cy-INV. Zur Klonierung der Fragmente B und C wurde das Plasmid p35S-Cy-INV mit HindIII verdaut, das zurückstehende 3'-Ende mit DNA Polymerase (Klenow Fragment) aufgefüllt, das Plasmid mit Asp 718 partiell verdaut und das die beiden intakten Fragmente B und C enthaltende Fragment mittels Gelelektrophorese von anderen Fragmenten abgetrennt. Dieses Fragment wurde sodann zwischen die Asp718 und die mit DNA Polymerase partiell mit G + A aufgefüllte EcoRI Schnittstelle des Polylinkers von pUG118 kloniert. Durch die partielle Auffüllung wurde die HindIII Schnittstelle erhalten.

Das Plasmid p33-Cy-INV wurde in analoger Verfahrensweise wie im Beispiel 1 beschrieben in die Pflanze eingeführt.

## Ausführungsbeispiel 6

Herstellung des Plasmids p355-V-INV und Einführung des Plasmids in das pflanzliche Genom des Tabaks

In analoger Weise, wie unter Beispiel 1 beschrieben, jedoch mit der Modifikation, daß vor die kodierende Sequenz des Invertase-Gens ein für die Direktion des Invertaseproteins in die Vakuolen notwendiges Peptid eines pflanzlichen Gens (Patatin-Gen pgT 5 aus Kartoffel, Rosahl et al. , Mol. General Genetics 203, 214-220) fusioniert wurde, wurde das Plasmid p35S-V-INV hergestellt. Das Plasmid p35S-V-INV hat eine Größe von 6,3 kb und besteht aus den drei Fragmenten A, B und C, die in die angegebenen Schnittstellen des Polylinkers von pUC 18 kloniert wurden (siehe Fig. 6) Fragment A (529 bp) beinhaltet den 35S Promotor des Cauliflower-Mosaik-Virus (CaMV). Das Fragment umfaßt die Nukleotide 6909 bis 7437 des CaMV (Franck et al., Cell 21, 285-2941). und wurde als Eco RI-Kpn I-Fragment aus dem Plasmid pDH5I (Pietrzak et al., Nucleic Acid Research 14, 5857-5868) isoliert und zwischen die Eco RI-Kpn I-Schnittstellen des Polylinkers des Plasmids pUC 18 kloniert (siehe Fig. 6).

Fragment B enthält die Nukleotide 707 bis 1895 der Sequenz des genomischen Patatinklons pgT5 (Rosahl et al., 1986), welches über einen Linker mit der Sequenz AGCTTTC an das suc 2 Gen aus Hefe, welches die Nukleotide +64 bis +1765 (Taussig und Carlson, (1983) Nucleic Acid Res. 11, 1943-1954) umfaßt, fusioniert ist. Hiermit wird ein die Direktion von Proteinen in die Vakuole höherer Pflanzen bewirkendes Peptid mit entsprechenden vakuolären Targetting-Signalen N-terminal an die Invertasesequenz fusioniert. Das Fragment B wurde als Eco RV-Xmn I-Fragment zwischen die SmaI/Pst I Schnittstellen des Polylinkers von pUC 18 gesetzt (siehe Fig. 6), wobei vor der Ligation die 3'-überhängenden Enden der Pst I Schnittstelle durch Inkubation mit T4-DNA-Polymerase stumpf gemacht worden waren.

Fragment C (192 bp) beinhaltet das Polyadenylierungssignal des Gens 3 der T-DNA des Ti-Plasmids pTiACH5 (Gielen et al., EMBO J. 3, 835-846) mit den Nukleotiden 11749-11939, welches als Pvu II-Hind III Fragment aus dem Plasmid pAGV 40 (Herrera-Estrella et al. (1983) Nature 303, 209-213) isoliert worden ist und nach Addition von Sph I Linkern an die Pvu II Schnittstelle zwischen die Sph I-Hind III Schnittstellen des Polylinkers von pUC 18 kloniert worden war.

Die Analyse der erhaltenen transgenen Tabakpflanzen mittels Invertase-Aktivitätstest zeigte, daß die vom suc 2 Gen kodierte Invertase nunmehr in der Vakuole lokalisiert ist (siehe Fig. 9). Transgene Tabakpflanzen zeigten zusätzlich zu einer Stauchung der Pflanzen einen neuen Blatt-Phänotyp. Dieser äußert sich darin, daß sich bei älteren Blättern, von der Blattspitze beginnend, chlorotische Bereiche entwickelten.

## Patentansprüche

1. Ein Plasmid, enthaltend eine DNA-Sequenz, dessen von dieser Sequenz kodiertes Produkt die Verteilung und/oder Bildung von Photoassimilaten in Pflanzen verändert und damit zu Veränderungen im Habitus und/oder Ertrag von Pflanzen führt.

2. Ein Plasmid gemäß Anspruch 1, dadurch gekennzeichnet, daß die DNA-Sequenz die DNA-Sequenz eines Gens ist, das für ein Saccharose-modifizierendes Enzym kodiert.

3. Ein Plasmid gemäß Anspruch 2, dadurch gekennzeichnet, daß die DNA-Sequenz die DNA-Sequenz eines Invertase-Gens ist.

4. Ein Plasmid gemäß Anspruch 3, dadurch gekennzeichnet, daß die DNA-Sequenz die DNA-Sequenz des Invertase-Gens suc2 aus Hefe ist.

5. Ein Plasmid gemäß den Ansprüchen 3 und 4, dadurch gekennzeichnet, daß die DNA-Sequenz des Invertase-Gens an die regulatorischen Regionen anderer Gene, die eine Expression des Invertase-Gens in pflanzlichen Zellen und Pflanzen sicherstellen, sowie gegebenenfalls an eine DNA-Sequenz, die die Direktion des Invertase-Proteins in Vakuolen der Pflanze und pflanzlichen Zellen sicherstellt, fusioniert ist.

6. Ein Plasmid gemäß Anspruch 5, dadurch gekennzeichnet, daß die regulatorischen Regionen Promotoren und Terminationssignale von pflanzlichen Genen sind.

7. Ein Plasmid gemäß Anspruch 6, dadurch gekennzeichnet, daß der Promotor der Promotor der 35 S RNA des Cauliflower-Mosaik-Virus ist.

8. Ein Plasmid gemäß Anspruch 6, dadurch gekennzeichnet, daß der Promotor der Promotor des Klasse I Patatin Gens B 33 ist.

9. Ein Plasmid gemäß Anspruch 5, dadurch gekennzeichnet, daß die DNA-Sequenz, die die Direktion des Invertase-Proteins in Vakuolen der Pflanze und pflanzlichen Zellen sicherstellt, eine DNA-Sequenz des Patatin-Gens pgT5 aus Solanum tuberonum der Nukleotid-Positionen +707 bis +1895 ist.

10. Ein Plasmid gemäß Anspruch 4, dadurch gekennzeichnet, daß die DNA-Sequenz des Invertase Gens suc2 an die regulatorischen Regionen anderer Gene, die eine Expression des Invertase-Gens in pflanzlichen Zellen und Pflanzen sicherstellen, sowie an eine der DNA-Sequenz des Invertase-Gens vorangestellten DNA-Sequenz einer für die Aufnahme in das endoplasmatische Retikulum von Pflanzen und Pflanzenzellen notwendigen Signalpeptids fusioniert ist.

11. Ein Plasmid gemäß Anspruch 10, dadurch gekennzeichnet, daß die regulatorischen Regionen Promotoren und Terminationssignale von pflanzlichen Genen sind.

12. Ein Plasmid gemäß Anspruch 11, dadurch gekennzeichnet, daß der Promotor der Promotor des ST-LS1 Gens ist.

13. Ein Plasmid gemäß Anspruch 11, dadurch gekennzeichnet, daß der Promotor der Promotor des Klasse I Patatin Gens B 33 ist.

14. Ein Plasmid gemäß Anspruch 11, dadurch gekennzeichnet, daß der Promotor der Promotor der 35S RNA des Cauliflower-Mosaik-Virus ist.

15. Ein Plasmid gemäß Anspruch 10, dadurch gekennzeichnet, daß die DNA-Sequenz des Signalpeptids vom Proteinase-Inhibitor II Gen aus Solanum tuberosum stammt.

16. Ein Plasmid gemäß den Ansprüchen 6 und 11, dadurch gekennzeichnet, daß das Terminationssignal das 3'-Ende der Poly-A-Seite des Octopin-Synthase Gens enthält.

17. Plasmid p 35 S - Cy-INV (DSM 5785)

18. Plasmid p 35 S - CW-INV (DSM 5788)

19. Plasmid p 33 - CW-INV (DSM 5787)

20. Plasmid pI 700 - CW-INV (DSM 5789)

21. Plasmid p 33 - Cy-INV (DSM 5786)

22. Plasmid p35S-V-INV (DSM 6142)

23. Verwendung der Plasmide p 35 S - Cy-INV, p 35 S - CW-INV, p 33 - CW-INV, pI 700 - CW-INV, p 33 - Cy-INV und p35S-V-INV zur Herstellung von im Habitus und Ertrag veränderten transgenen Pflanzen.

24. Eine Pflanze, enthaltend eine DNA-Sequenz, dessen von dieser Sequenz kodiertes Produkt die Verteilung und/oder Bildung von Photoassimilaten verändert und damit zu Veränderungen im Habitus und/oder Ertrag führt.

25. Eine Pflanze gemäß Anspruch 24, dadurch gekennzeichnet, daß die DNA-Sequenz die DNA-Sequenz eines Gens ist, das für ein Saccharose-modifizierendes Enzym kodiert.

26. Eine Pflanze gemäß Anspruch 25, dadurch gekennzeichnet, daß die DNA-Sequenz die DNA-Sequenz eines Invertase-Gens ist.

27. Eine Pflanze gemäß Anspruch 26, dadurch gekennzeichnet, daß die DNA-Sequenz die DNA-Sequenz des Invertase-Gens suc2 aus Hefe ist.

28. Eine Pflanze gemäß den Ansprüchen 26 und 27, dadurch gekennzeichnet, daß die DNA-Sequenz des Invertase-Gens an die regulatorischen Regionen anderer Gene, die eine Expression des Invertase-Gens in pflanzlichen Zellen und Pflanzen sicherstellen, sowie gegebenenfalls an eine DNA-Sequenz, die die Direktion des Invertase-Proteins in Vakuolen der Pflanze und pflanzlichen Zellen sicherstellt, fusioniert ist.

29. Eine Pflanze gemäß Anspruch 28, dadurch gekennzeichnet, daß die regulatorischen Regionen Promotoren und Terminationssignale von pflanzlichen Genen sind.

30. Eine Pflanze gemäß Anspruch 29 dadurch gekennzeichnet, daß der Promotor der Promotor der 35 S RNA des Cauliflower-Mosaik-Virus ist.

31. Eine Pflanze gemäß Anspruch 29, dadurch gekennzeichnet, daß der Promotor der Promotor des Klasse I Patatin Gens B 33 ist.

32. Eine Pflanze gemäß Anspruch 28, dadurch gekennzeichnet, daß die DNA-Sequenz, die die Direktion des Invertaseproteins in Vakuolen der Pflanze und pflanzlichen Zellen sicherstellt, eine DNA-Sequenz des Patatin-Gens pgT5 aus Solanum tuberosum der Nukleotid-Positionen +707 bis +1895 ist.

33. Eine Pflanze gemäß Anspruch 27, dadurch gekennzeichnet, daß die DNA-Sequenz des Invertase Gens suc2 an die regulatorischen Regionen anderer Gene, die eine Expression des Invertase-Gens in pflanzlichen Zellen und Pflanzen sicherstellen, sowie an eine der DNA-Sequenz des Invertase-Gen vorangestellten DNA-Sequenz einer für die Aufnahme in das endoplasmatische Retikulum von Pflanzen und Pflanzenzellen notwendigen Signalpeptids fusioniert ist.

34. Eine Pflanze gemäß Anspruch 33, dadurch gekennzeichnet, daß die regulatorischen Regionen Promotoren und Terminationssignale von pflanzlichen Genen sind.

35. Eine Pflanze gemäß Anspruch 34, dadurch gekennzeichnet, daß der Promotor der Promotor des ST-LS1 Gens ist.

36. Eine Pflanze gemäß Anspruch 34, dadurch gekennzeichnet, daß der Promotor der Promotor des Klasse I Patatin Gens B 33 ist.

37. Eine Pflanze gemäß Anspruch 34, dadurch gekennzeichnet, daß der Promotor der Promotor der 35 S RNA des Cauliflower-Mosaik-Virus ist.

38. Eine Pflanze gemäß Anspruch 33, dadurch gekennzeichnet, daß die DNA-Sequenz des Signalpeptids vom Proteinase-Inhibitor II Gen aus Solanum tuberosum stammt.

39. Eine Pflanze gemäß den Ansprüchen 29 und 34, dadurch gekennzeichnet, daß das Terminationssignal das 3'-Ende der Poly-A-Seite des Octopin-Synthase Gens enthält.

40. Eine Pflanze gemäß den Ansprüchen 24-39, dadurch gekennzeichnet, daß es eine Nutzpflanze ist.

41. Eine Pflanze gemäß Anspruch 40, dadurch gekennzeichnet, daß es sich um eine Tabak-, Kartoffel-, Tomaten-, Zuckerrüben-, Sojabohnen-, Gersten-, Weizen-, Mais- oder Zuckerrohrpflanze handelt.

42. Verwendung der Plasmide gemäß Anspruch 23 zur Herstellung von Pflanzen mit größerem Trockengewicht der Samen, Früchte, Rüben und Knollen.

43. Verwendung der Plasmide gemäß Anspruch 23 zur Herstellung von Kartoffelpflanzen mit größeren Knollen und größerem Knollentrockengewicht.

44. Pflanzen und pflanzliche Produkte gemäß den Ansprüchen 41 und 43 zur Erhöhung des Ernteertrages.

45. Eine Pflanzenzelle oder Pflanzen, enthaltend eine DNA-Sequenz, bei dem ein Teil dieser DNA-Sequenz für einen Teil eines Fusionsproteins kodiert, bei dem dieser Teil für die Direktion des Fusionsproteins in die Vakuolen sorgt.

46. Eine Pflanzenzelle oder Pflanzen gemäß Anspruch 45, dadurch gekennzeichnet, daß die DNA-Sequenz des den Transport in Vakuolen bewirkende Peptids vom Patatin-Gen aus Solanum tuberosum stammt.

47. Eine Pflanzenzelle oder Pflanzen gemäß Anspruch 46, dadurch gekennzeichnet, daß der vom Patatin-Gen stammende Teil der DNA-Sequenz die Nukleotide Position 707 bis 1895 enthält.

Fig. 1

Fig. 2

Fig. 3

Fig. 4

[DraI/SmaI]

HindIII SphI PstI SalI Xbal BamI   Asp718   [XmnI/PstI]SphI   [HindIII/EcoRI]

A   B   C

[DraI/SmaI]   1526 bp   1726 bp   192 bp

p 33 - Cy - INV   6,8 kb

Fig. 5

Eco RI   KpnI   [SmaI/EcoRV]   [XmnI/PstI]   SphI   Hind III

A   B   C

529 bp   2898 bp   192 bp

p 35 S - V - INV   6,3 kb

Fig. 6

Fig. 7

Fig. 8

Fig. 9